# EUROPÄISCHE PATENTSCHRIFT

(11) **EP 0 593 992 B1**
(45) Veröffentlichungstag und Bekanntmachung des Hinweises auf die Patenterteilung: **23.07.1997**
(21) Anmeldenummer: 93116287.9
(22) Anmeldetag: 08.10.1993
(51) Int. Cl.: G01N 33/18

(54) **Vorrichtung zur Überwachung von Trinkwasser oder gereinigtem Abwasser**
Apparatus for monitoring drinking water or purified waste water
Appareil pour contrôler l'eau potable ou des eaux usées purifiées

(30) Priorität: 12.10.1992 DE 4234371
(43) Veröffentlichungstag der Anmeldung: 27.04.1994
(73) Patentinhaber: GRUNDIG Aktiengesellschaft, 90762 Fürth (DE)
(72) Erfinder: Hallas, Ernst, Dr. rer. nat., GRUNDIG E.M.V., D-90748 Fürth (DE)

(56) Entgegenhaltungen:
- EP-A- 0 162 668
- DE-A- 4 110 877
- FR-A- 2 356 141
- FR-A- 2 573 875
- ZEITSCHRIFT FüR WASSER- UND ABWASSER-FORSCHUNG Bd. 22 , 1989 , WEINHEIM, DEUTSCHLAND Seiten 120 - 124 H. PETRY 'Automatisiertes Frühwarnsystem zur kontinuierlichen Gewässerkontrolle mit Tubificiden als Schadstoffindikatoren'

## Beschreibung

Die Erfindung betrifft eine Vorrichtung zur Überwachung von Trinkwasser oder gereinigtem Abwasser auf Schadstoffe nach dem Oberbegriff des Anspruches 1.

Zum Nachweis von Schadstoffen in Gewässern ist es weit verbreitet, Proben zu entnehmen und seine Inhaltsstoffe, insbesondere die Schadstoffe, mittels chemischer oder fotometrischer Analyse zu bestimmen. Hierbei erhält man eine definitive Aussage über die Konzentration der Schadstoffe. Die Auswertung kann jedoch häufig nicht vor Ort stattfinden, so daß die Zeit zwischen der Probenentnahme und dem Erhalt eines Analyseergebnisses für eine schnelle Reaktion bei Vorliegen eines Schadstoffes zu groß ist.

Bei der Überwachung des Trinkwassers oder von gereinigten Abwässern ist es notwendig, das Vorhandensein von Schadstoffen sofort zu erkennen und erste Maßnahmen zu ergreifen, wobei es meist nicht notwendig ist, sofort ein genaues Analyseergebnis in Bezug auf Art und Konzentration des Schadstoffes zu erhalten.

Für eine pauschale Erkennung von Schadstoffen in Gewässern sind verschiedene biologische Tests bekannt, bei denen die Bewegung von Fischen beobachtet wird.

Bessere Ergebnisse liefert die Bewegungsbeobachtung von Schlammröhrenwürmern. Eine Vorrichtung zur Durchführung von Bewegungstests mit Schlammröhrenwürmern ist in einem Aufsatz von Prof. Dr. Dr. habil. H. Petry, "Automatisiertes Frühwarnsystem zur kontinuierlichen Gewässerkontolle mit Tubificiden als Schadstoffindikatoren", Z. Wasser-Abwasser-Forsch., Heft 22, 1989, Seiten 120-124 beschrieben.

Die Versuchsanordnung besteht aus einem Testgefäß zur Aufnahme der Versuchstiere, einem Videosystem zur Registrierung der Tierbewegungen und einem Computersystem zur Verarbeitung der Meßdaten.
Bei dieser bekannten Anordnung werden die Schlammröhrenwürmer in das Testgefäß eingebracht und mit einer Videokamera überwacht. Zunächst wird durch das Testgefäß schadstofffreies Wasser geleitet. Nach kurzer Zeit bilden die Schlammröhrenwürmer ein Knäuel, das nur geringe Bewegungsaktivität zeigt. Diese Bewegungsaktivität wird mit der Videokamera und einem daran angeschlossenen Videocontroller erfasst und die mittlere Bewegungsaktivität ermittelt.

Anschließend wird dem Testgefäß das zu untersuchende Wasser zugeführt. Während des Tests wird die mittlere Bewegungsaktivität der Versuchstiere beobachtet und ausgewertet. Befinden sich in dem zu untersuchenden Wasser Schadstoffe, reagieren die Schlammröhrenwürmer mit erhöhter Bewegungsaktivität, die zur Alarmgabe führt.

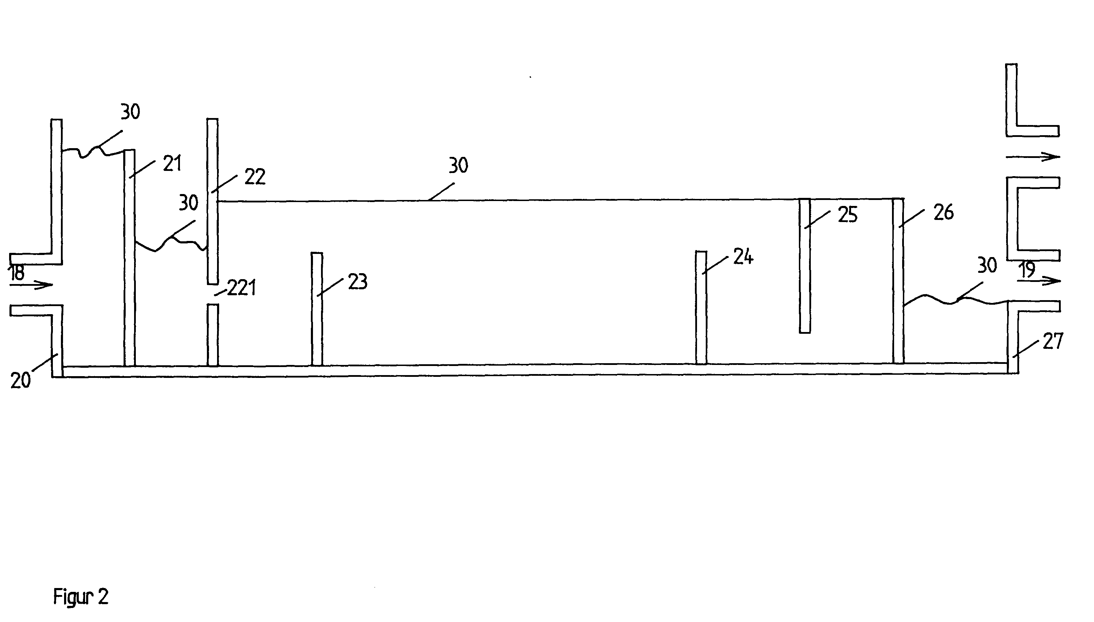

Das verwendete Testgefäß besteht im wesentlichen aus einem Wassereinlaufteil, der Meßzelle und dem Wasserauslaufteil, wie in Figur 2 dargestellt. Bei der bekannten Anordnung handelt es sich um ein rechteckiges Gefäß mit glattem Boden aus Plexiglasplatten.
Durch den Einlauf 18 an der äußeren Gefäßwand 20 wird das zu untersuchende Wasser in eine Vorkammer geleitet, die durch einen Steg 21 mit der äußeren Gefäßwand gebildet wird. Über den Steg 21 tritt das Wasser in den nächsten Gefäßabschnitt, der durch den Steg 22 begrenzt wird. Im Steg 22 sind Bohrungen 221 angebracht, durch die das Wasser in die nächste Kammer tritt, die durch den Steg 23 begrenzt wird. Die nachfolgende Meßzelle wird durch die Stege 23 und 24 begrenzt, die niedriger sind als die Wasseroberfläche 30.
Im Auslaßteil befinden sich noch zwei Stege 25 und 26, die die Höhe der Wasseroberfläche 30 bestimmen.
Das Wasser wird schließlich durch den Auslauf 19 abgeführt. Die einzelnen Stege, insbesondere die Stege 22 und 25 dienen dazu, die Wasseroberfläche glatt zu halten, da Wellenbewegungen auf der Wasseroberfläche von der Videoauswertung als Bewegungen interpretiert werden und somit Fehlalarm auslösen können.

Bei Versuchen hat sich gezeigt, daß nach einer Schadstoffeinleitung, also nachdem sich die Schlammröhrenwürmer in der Meßzelle verteilt hatten, sich oftmals zwei Knäuel gebildet haben, nachdem wieder sauberes Wasser zugeführt wurde. Weiterhin nahm in den Ecken der Meßzelle die Bewegungsaktivität der Schlammröhrenwürmer beim Einleiten von sauberem Wasser nicht ab, da in den Ecken das schadsoffbelastete Wasser nicht ausgespült wurde.

Es ist daher Aufgabe der Erfindung, eine Vorrichtung der eingangs genannten Art anzugeben, bei der eine gute Durchspülung der Meßzelle gegeben ist und bei der sich das Knäuel aus Schlammröhrenwürmern auch nach hoher Bewegungsaktivität nicht aufteilt.

Diese Aufgabe wird bei einer Vorrichtung mit den im Oberbegriff des Anspruchs 1 enthaltenen Merkmalen durch die kennzeichnenden Merkmale des Anspruchs 1 gelöst.

Bei der erfindungsgemäßen Vorrichtung ist das Wassertestbecken rotationssymmetrisch ausgebildet. Der Wasserzulauf erfolgt im äußeren Wasserzulaufteil des Wassertestbeckens an mindestens einer Stelle, wobei die Verwendung von mehreren Stellen, an denen das Wasser zuläuft, eine gleichmäßigere Verteilung des zu untersuchenden Wassers erlaubt.
Der Wasserablauf befindet sich im Boden der Meßzelle, wobei die Meßzelle einen trichterförmigen Verlauf zum Wasserablauf hin aufweist. In der Meßzelle selbst ist ein schalenförmiger Träger für die Versuchstiere unterhalb des Wasserspiegels eingebracht.

Die erfindungsgemäße Vorrichtung hat den Vorteil, daß durch die rotationssymmetrische Anordnung des Wassertestbeckens, insbesondere bei mehreren Einlaufstellen und dem Wasserabfluß in der Mitte der Meßzelle, eine gleichmäßige Beströmung erfolgt, die sich zum Ablauf hin verstärkt. Somit bildet sich eine Strömung in der Meßzelle aus, die eine gleichmäßige Durchflutung gewährleistet, so daß keine Schadstoffansammlung erfolgt. Im weiteren wird durch den schalenförmigen Träger für die Versuchstiere erreicht, daß sie im tiefsten Punkt der Schale zentriert werden, sobald sie nicht die eigene Bewegungsenergie, die sie im Fall der Schadstoffeinleitung entwickeln, auseinandertreibt.

Eine vorteilhafte Ausgestaltung der Erfindung sieht vor, daß im Bereich der Meßzelle eine ringförmige Abdeckung angebracht ist, die nur den von der Aufnahmekamera erfassten Bereich frei läßt.
Durch diese Abdeckung wird die Wasseroberfläche zusätzlich ruhig gehalten, um Störungen bei der Bildauswertung durch eine unruhige Wasseroberfläche zu vermeiden. Weiterhin wird die Strömung in der Meßzelle günstig beeinflußt, so daß besonders der schalenförmige Träger gut durchspült wird.

Eine weitere Verbesserung in Bezug auf die Strömung läßt sich erreichen, wenn die ringförmige Abdeckung nach unten hin trichterförmig zuläuft.

Für die Aufnahme mit der Videokamera und die nachfolgende Auswertung hat es sich als günstig erwiesen, den Boden der Meßzelle aus durchsichtigem oder optisch trübem Material anzufertigen. Insbesondere werden hierbei Vorteile erzielt, wenn am Boden der Meßzelle eine Beleuchtung angebracht ist. Durch die Beleuchtung von unten erhält man kontrastreichere Bilder, die bei der Bewertung der Bewegungsaktivität leichter auszuwerten sind.

In einer weiteren vorteilhaften Ausgestaltung der Erfindung wird vorgesehen, daß sich am Wasserablauf am Boden der Meßzelle ein Siphon anschließt, wobei die Höhe des Siphonauslaufes die Füllstandshöhe in der Meßzelle bestimmt.

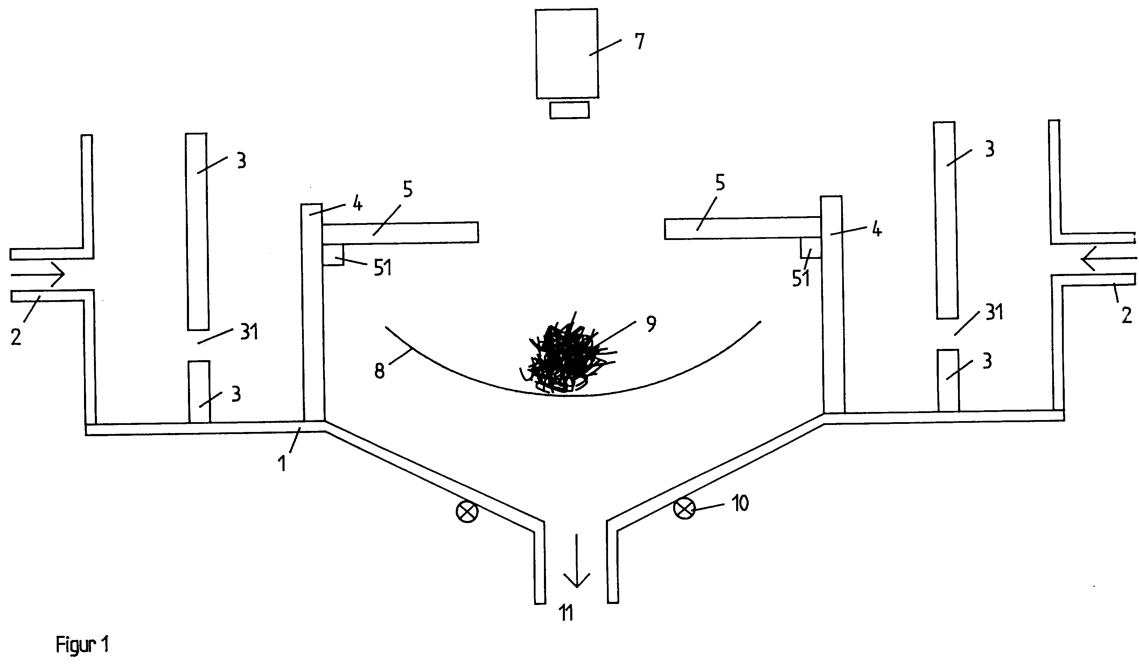

Im folgenden wird die Erfindung an Hand einer Ausführungsform nach Figur 1 näher erläutert.

Diese Figur zeigt einen Querschnitt eines Wassertestbeckens gemäß der Erfindung. Dieses Wassertestbecken ist rotationssymmetrisch aufgebaut und enthält zwei grobe Unterteilungen, und zwar den äußeren Wassereinlaufbereich und den Bereich der Meßzelle. Der äußere Wassereinlaufbereich wird von der Außenwand des Wassertestbeckens und dem Steg 4 begrenzt. An der Außenwand des Wassertestbeckens befinden sich die Wassereinläufe 2, die an der Außenwand in gleichen Abständen angeordnet sind.
Im äußeren Bereich ist ein Steg 3 angeordnet, der Löcher 31 enthält, die gleichmäßig über den Steg 3 verteilt angeordnet sind.
Durch die Anordnung des Wassereinlaufbereiches wird erreicht, daß die Wasseroberfläche ruhig bleibt und keine Wellenbewegungen auftreten.
Über den Steg 4 tritt das Wasser in den Bereich der Meßzelle ein. Zur Stabilisierung der Wasseroberfläche ist eine ringförmige Abdeckung 5 vorgesehen, die auf die Halterung 51 aufgelegt ist. Die ringförmige Abdeckung 5 ist in der Mitte so weit offen, daß mit der Videokamera 7 die Versuchstiere 9 auf dem schalenförmigen Träger 8 beobachtet werden können.

Der Boden 1 des Wassertestgefäßes besteht aus durchsichtigem oder trübem Material und ist im Bereich des Wasserzulaufes eben, während er im Bereich der Meßzelle trichterförmig zum Auslauf 11 hin zuläuft. Unter dem Boden 1 ist im Bereich der Meßzelle eine vorzugsweise ringförmige Beleuchtung 10 angebracht, um den Kontrast bei der Bildaufnahme zu steigern.

## Patentansprüche

1. Vorrichtung zur Überwachung von Trinkwasser oder gereinigtem Abwasser auf Schadstoffe durch Überwachung der Bewegungen von wirbellosen Versuchstieren, insbesondere Schlammröhrenwürmern, mit einer Aufnahmekamera, einem digitalen Videocontroller, einem Monitor, einem Alarmgeber und einem Wassertestbecken, wobei das Wassertestbecken aus einem äußeren Wasserzulaufteil und einer inneren Meßzelle besteht,
**dadurch gekennzeichnet,** daß
- das Wassertestbecken rotationssymmetrisch ausgebildet ist,
- im Wasserzulaufteil des Wassertestbeckens an der Außenwand mindestens ein Wasserzulauf angeordnet ist,
- der Wasserablauf im Boden der Meßzelle angeordnet ist,
- die Meßzelle zum Wasserablauf hin einen trichterförmigen Verlauf hat, und
- in der Meßzelle eine schalenförmige Einrichtung unterhalb des Wasserspiegels angeordnet ist, in der die Versuchstiere eingebracht sind.

2. Vorrichtung nach Anspruch 1, **dadurch gekennzeichnet,** daß
im Bereich der Meßzelle eine ringförmige Abdeckung angebracht ist, die nur den von der Aufnahmekamera erfassten Bereich freiläßt.

3. Vorrichtung nach Anspruch 2, **dadurch gekennzeichnet**, daß
die ringförmige Abdeckung zur Mitte hin trichterförmig nach unten verläuft.

4. Vorrichtung nach einem oder mehreren der Ansprüche 1 bis 3, **dadurch gekennzeichnet**, daß
der Boden der Meßzelle aus durchsichtigem Material ausgeführt ist.

5. Vorrichtung nach einem oder mehreren der Ansprüche 1 bis 3, **dadurch gekennzeichnet,** daß der Boden der Meßzelle aus optisch trübem Material ausgeführt ist.

6. Vorrichtung nach Anspruch 4 oder 5, **dadurch gekennzeichnet,** daß
am Boden der Meßzelle eine ringförmige Beleuchtungsvorrichtung angebracht ist.

7. Vorrichtung nach einem oder mehreren der Ansprüche 1 bis 6, **dadurch gekennzeichnet,** daß sich am Wasserablauf ein Siphon anschließt, das derart ausgeführt ist, daß es die Füllstandshöhe in der Meßzelle bestimmt.

## Claims

1. Apparatus for monitoring drinking water or purified waste water for pollutants by monitoring the movements of invertebrate test animals, in particular sabellids, with a camera, a digital video controller, a monitor, an alarm generator and a water test vessel, wherein the water test vessel consists of an outer water feed part and an inner measuring cell, characterised in that
- the water test vessel is formed so as to be rotationally symmetrical,
- at least one water feed is arranged at the outer wall in the water feed part of the water test vessel,
- the water drain is arranged in the bottom of the measuring cell,
- the measuring cell extends towards the water drain in the shape of a funnel, and
- a dish-shaped device, in which the test animals are placed, is arranged below the surface of the water in the measuring cell.

2. Apparatus according to claim 1, characterised in that an annular cover is fitted in the region of the measuring cell and only leaves the region recorded by the camera free.

3. Apparatus according to claim 2, characterised in that the annular cover extends downwards towards the centre in the shape of a funnel.

4. Apparatus according to one or more of claims 1 to 3, characterised in that the bottom of the measuring cell is made from a transparent material.

5. Apparatus according to one or more of claims 1 to 3, characterised in that the bottom of the measuring cell is made from an opaque material.

6. Apparatus according to claim 4 or 5, characterised in that an annular lighting apparatus is fitted at the bottom of the measuring cell.

7. Apparatus according to one or more of claims 1 to 6, characterised in that a siphon adjoins the water drain and is formed such that it determines the level in the measuring cell.

## Revendications

1. Dispositif pour contrôler la teneur en substances polluantes de l'eau potable et des eaux usées purifiées par la surveillance des mouvements d'animaux de laboratoire invertébrés, notamment des vers tubulaires de vase, comprenant une caméra de prise de vue, un contrôleur de vidéo numérique, un moniteur, un dispositif de déclenchement d'alarme et un bassin d'essai d'eau, le bassin d'essai d'eau étant constitué d'une partie externe d'arrivée d'eau et d'une cellule de mesure interne, caractérisé en ce que le bassin d'essai d'eau est réalisé de façon symétrique en rotation,
- dans la partie d'arrivée d'eau du bassin d'essai d'eau, au moins une arrivée d'eau est agencée à la paroi externe, la sortie d'eau étant agencée dans le fond de la cellule de mesure,
- la cellule de mesure possède, en direction de la sortie de l'eau, une forme d'entonnoir, et
- dans la cellule de mesure,un agencement en forme de cuvette est agencé en dessous du niveau d'eau, dans lequel les animaux de laboratoire sont introduits.

2. Dispositif selon la revendication 1, caractérisé en ce qu'une couverture en forme d'anneau est agencée dans la zone de la cellule de mesure qui ne laisse libre que la zone saisie par la caméra de prise de vue.

3. Dispositif selon la revendication 2, caractérisé en ce que la couverture en forme d'anneau s'étend en direction du centre selon une forme d'entonnoir vers le bas.

4. Dispositif selon l'une ou plusieurs des revendications 1 à 3, caractérisé en ce que le fond de la cellule de mesure est réalisé en un matériau transparent.

5. Dispositif selon l'une ou plusieurs des revendications 1 à 3, caractérisé en ce que le fond de la cellule de mesure est réalisé en un matériau terne.

6. Dispositif selon la revendication 4 ou 5, caractérisé en ce qu'au fond de la cellule de mesure est agencé un dispositif d'éclairage en forme d'anneau.

7. Dispositif selon l'une ou plusieurs des revendications 1 à 6, caractérisé en ce qu'à la sortie d'eau s'ensuit un siphon qui est réalisé de telle sorte qu'il commande le niveau de remplissage dans la cellule de mesure.
